# EUROPÄISCHE PATENTSCHRIFT

(11) **EP 2 103 280 B1**
(45) Veröffentlichungstag und Bekanntmachung des Hinweises auf die Patenterteilung: **21.04.2010**
(21) Anmeldenummer: 09003856.3
(22) Anmeldetag: 18.03.2009
(51) Int. Cl.: A61F 2/18

(54) **Gehörknöchelchenprothese mit variablen Ankopplungsflächen**
Ossicular prosthetic with variable coupling surfaces
Prothèse de l'osselet de l'oreille dotée de surfaces d'accouplement variables

(30) Priorität: 20.03.2008 DE 102008015117
(43) Veröffentlichungstag der Anmeldung: 23.09.2009
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus

(56) Entgegenhaltungen:
- DE-B3-102007 013 708
- US-A1- 2004 162 614
- US-A1- 2006 271 190

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein im Wesentlichen plattenförmiges erstes Befestigungselement zur Anlage am Trommelfell oder an der Steigbügelfußplatte und an ihrem anderen Ende ein zweites Befestigungselement zur mechanischen Verbindung mit einem Glied oder Teilen eines Gliedes der Gehörknöchelchenkette oder mit dem Innenohr sowie ein die beiden Befestigungselemente Schall leitend miteinander verbindendes Verbindungselement umfasst, und wobei das plattenförmige erste Befestigungselement einen radial inneren Ankoppelbereich zum mechanischen Ankoppeln des ersten Befestigungselements an das Verbindungselement sowie mehrere Stegelemente zur radialen Verbindung des radial inneren Ankoppelbereichs mit radial äußeren Abschnitten des ersten Befestigungselements aufweist.

Eine derartige Vorrichtung ist bekannt aus der DE 10 2007 013 708 B3.

Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung bzw. Signalübertragung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Nachdem die Prothese operativ im Mittelohr platziert wurde und das Trommelfell wieder verschlossen ist, beginnt die so genannte Einheilungsphase. In dieser Zeit bilden sich Narben und Gewebestränge und diese verursachen unvorhersehbare Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse postoperative Mobilität und Flexibilität aufweist, so dass sich die Kopfplatte nach der Operation selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells von Mensch zu Mensch variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen bekannt, die elastische Teile und/oder Gelenke aufweisen. Eine solche gelenkige Verbindung zwischen einem an der Steigbügelfußplatte montierbaren Befestigungselement und dem länglichen Schaft ist in der EP 1 181 907 B1 beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einher gehende akute oder chronische Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sog. Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet und meistens als länglicher Schaft ausgeführt ist. Eine solche in sich starre, aber gegenüber dem Verbindungselement verkippbare Kopfplatte ist u.a. beschrieben in der US 2004/0162614 A1, in dem Artikel M.W. YUNG, Ph.D., F.R.C.S., D.L.O., C. BREWIS, F.R.C.S., "A comparison of the user-friendliness of hydroxyapatite and titanium ossicular prostheses", The Journal of Laryngology & Otology, February 2002, Vol. 116, pp. 97-102, oder beispielsweise auch in der US 2006/0271190 A1.

Nachteilig bei diesen bekannten Gehörknöchelchenprothesen ist allerdings, dass durch die in sich starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Um eine hohe postoperative Flexibilität und Variabilität der Prothese zu erreichen, wobei gleichzeitig die Qualität der Schallleitung durch die Prothese erheblich erhöht werden soll, ohne dass es zu den oben geschilderten Komplikationen kommen kann, schlägt die eingangs zitierte DE 10 2007 013 708 B3 vor, dass die Stegelemente geometrisch so gestaltet sind, dass sie bei lokaler Medialbewegung des Trommelfells dieser Medialbewegung lokal folgen, jedoch die Bewegung nicht an entfernte Bereiche der Kopfplatte weitergeben. Durch diese flexible Gestaltung der Gehörknöchelchenprothese wird bei einer solchen kleineren Medialbewegung des Trommelfells eine starre Verkippung der gesamten Kopfplatte vermieden. Vielmehr verwindet sich die Kopfplatte lokal in sich selbst, wobei sie jedoch bei großflächigen, durch Schall verursachten Bewegungen des Trommelfells diese an das Verbindungselement weitergibt, so dass eine optimale Übertragung des Schalls bzw. des Schallsignals vom Trommelfell zum Mittelohrraum hin und weiter ans Innenohr gewährleistet wird.

Damit wird zwar gegenüber dem übrigen bekannten Stand der Technik eine ganz erhebliche Verbesserung erzielt. Leider bestehen jedoch noch weitere Probleme, die mit diesen Maßnahmen alleine nicht gelöst werden können:

Pathologie und Anatomie können im Rahmen einer Tympanoplastik im menschlichen Mittelohr ganz unterschiedliche, spezifisch vom individuellen Patienten abhängige Struktur-Rekonstruktionen notwendig machen. Je nach Ausmaß und Form von eventuell noch vorhandenen und vielleicht teilweise intakten Teilen der Mittelohranatomie, wie des Hammers (=Malleus), des Ambosses (=Incus), des Steigbügels (=Stapes) oder des Trommelfells, werden entsprechend viele, zum Teil ganz erheblich in Form und Größe voneinander abweichende Geometrien der einzusetzenden Mittelohrprothesen benötigt.

Da vor Beginn einer Mittelohr-Operation nur sehr schwer oder gar nicht vorhergesagt werden kann, wie sich die spätere Rekonstruktion des Trommelfells und der Gehörknöchelchenkette im Verlauf der Operation darstellen wird (wenn überhaupt, dann nur grob, aber praktisch niemals genau), müssen derzeit für jede aktuell durchzuführende Operation immer sehr viele Mittelohrprothesen mit unterschiedlichen Geometrien, Formen und Größen bereitgehalten werden, damit der Chirurg intraoperativ stets aus einer vorrätigen Vielfalt heraus die jeweils am besten passende Prothese auswählen kann, die es ihm ermöglicht, speziell auf die jeweilige Gegebenheit einzugehen. Ansonsten kann es dazu kommen, dass keine optimale Versorgung gewährleistet ist.

Hinzu kommt, dass die genannten intraoperativen Anpassungsprobleme der Gehörknöchelchenprothese nicht nur im Anlagebereich des ersten Befestigungselements an das Trommelfell auftreten können, sondern ebenso im Bereich eines gegebenenfalls erforderlichen, ebenfalls plattenförmigen zweiten Befestigungselements, das zur Anlage der Prothese auf der Steigbügelfußplatte dienen kann. Insbesondere für den zum Innenohr hin abschließenden Bereich einer Totalrekonstruktion weist eine Totalprothese zu diesem Zweck normalerweise einen Stempel mit einem Standard-Durchmesser von 0,8 mm auf. Oftmals wird von chirurgischer Seite aus der Wunsch geäußert, dass man gerne - je nach intraoperativer Situation - unterschiedliche Größenareale zur Verfügung hätte, die auf die Fußplatte des Steigbügels aufgesetzt werden können. Die Bereitstellung eines zusätzlichen, mit dem Stempel verbundenen oder verbindbaren Befestigungselements, welches hinsichtlich der Größe seiner Fläche in weiten Grenzen variabel wäre, würde diesem Wunsch der Fachwelt entgegen kommen.

Eine derartige Vorrichtung ist bekannt aus der US 2006/0271190 A, die die Merkmale das Oberbegriffs des Anspruch 1 zeigt. Falls die Gehörknöchelchenprothese keine Totalprothese und damit das erste Befestigungselement nicht als Kopfplatte zur Anlage am Trommelfell ausgebildet ist, sondern als Klammer zur Befestigung der Prothese an einem Glied der Gehörknöchelchenkette, treten die beschriebenen Anpassungsprobleme eventuell auch ausschließlich am Innenohrseitigen Ende der Gehörknöchelchenprothese auf.

Ein weiteres Problem liegt darin, dass - Weltweit gesehen - äußerst unterschiedliche chirurgische Techniken angewendet werden, welche verschiedenartige Rekonstruktionen im Mittelohr postulieren. Diese erfordern entsprechend angepasste, voneinander in Form und Größe sehr unterschiedliche Mittelohrprothesen, welche wiederum während jeder Operation vorrätig gehalten werden müssen, um dem Chirurgen die Möglichkeit zur geben, die nach seiner Einschätzung jeweils für den aktuell vorgefundenen Fall optimale Methode anzuwenden.

Aufgabe der vorliegenden Erfindung ist es demgegenüber, eine gattungsgemäße Mittelohrprothese der eingangs beschriebenen Art mit möglichst einfachen technischen Mitteln unaufwändig und kostengünstig dahin gehend zu verbessern, dass die Anzahl der intraoperativ bereit zuhaltenden unterschiedlichen Prothesen ganz erheblich verringert, vorzugsweise auf eine einzige Standard-Prothese reduziert werden kann, ohne dabei die Möglichkeit zur optimalen Adaption der Prothese im konkreten Einzelfall zu verlieren.

Erfindungsgemäß wird diese Aufgabe auf ebenso überraschend einfache wie wirkungsvolle Art und Weise dadurch gelöst, dass der Ankoppelbereich und/oder die Stegelemente und/oder die radial äußeren Abschnitte geometrisch so gestaltet sind, dass sie zusammen Teile eines Puzzles bilden, aus denen das plattenförmige erste Befestigungselement vollständig zusammengesetzt werden kann, wobei die einzelnen Puzzle-Teile knöpfbar aneinander gefügt oder auseinander genommen werden können, und wobei das erste Befestigungselement im zusammen geknöpften Zustand der Puzzle-Teile in sich mechanisch stabil ist.

Das plattenförmige erste Befestigungselement der erfindungsgemäßen Mittelohrprothese, welches z.B. als Kopfplatte ausgestaltet sein kann, die im Rahmen einer Tympanoplastik gegen das Trommelfell gelegt wird, ist so aufgebaut, dass es in seiner Form und Fläche in sehr weiten Grenzen variabel ist. Die standardmäßig vorrätig gehaltene erfindungsgemäße Prothese lässt sich daher intraoperativ in allen Belangen ganz einfach, sehr variabel und äußerst zielgenau so umgestalten, wie es die vorgefundene patientenspezifische Situation jeweils gerade erfordert.

Einfache, ad hoc vornehmbare Veränderungen der erfindungsgemäßen Standard-Prothese, etwa hinsichtlich von Winkeln, Längen oder Flächengrößen, dienen einer erheblich verbesserten Adaption im konkreten Einzelfall. Somit bietet die erfindungsgemäße MittelohrProthese dem Operateur ein extrem hohes Maß an Variabilität und Flexibilität, ohne dass dazu die bisher übliche Bevorratung einer großen Vielzahl unterschiedlichster Prothesenformen, -größen und -geometrien erforderlich ist. Der Chirurg kann damit intraoperativ an der Prothese gezielte Veränderungen vornehmen, die es ihm ermöglichen, die Prothese speziell auf die jeweilige Gegebenheit einzustellen bzw. anzupassen.

Durch die erfindungsgemäß angewandte Puzzletechnik ist es ganz leicht möglich, beispielsweise die Kopfplatte der Prothese in ihrer Größe so zu verändern, dass sie nur speziell gewollte Bereiche am Trommelfell berührt; in der Regel diejenigen, bei denen man genau weiß, dass sie für die akustische Übertragung eine wesentliche Rolle spielen.

Oftmals liegt der Fall vor, dass der Hammergriff am Trommelfell vorhanden ist - oder eben auch fehlt - je nach dem wie oft voroperiert, welche Operationsmethode angewendet und welche spezifische Maßnahme ergriffen wurde. Entsprechend kann bei einer erfindungsgemäß flächenvariabel gestalteten Kopfplatte durch den Operateur zielgenau und exakt richtig reagiert werden, also der für den Hammergriff verantwortliche Bereich abgenommen oder - je nach Bedarfangefügt werden.

Ähnliches gilt auch für eine Ankopplung der Gehörknöchelchenprothese an die Steigbügelfußplatte, wo ebenfalls durch die erfindungsgemäße flächenvariable Gestaltung des entsprechenden plattenförmigen Befestigungselements eine bisher nicht gekannte intraoperative Flexibilität im Hinblick auf eine optimale Anpassung an die individuelle Patientensituation geboten werden kann.

Vorzugsweise ist der radial innere Ankoppelbereich zum mechanischen Ankoppeln des ersten Befestigungselements an das Verbindungselement zentral um den Flächenschwerpunkt des plattenförmigen ersten Befestigungselements angeordnet.

Von der erfindungsgemäßen Grundidee kann in doppelter Weise profitiert werden, wenn beide Befestigungselemente plattenförmig gestaltet und aus Puzzle-Teilen aufgebaut sind, wobei das erste Befestigungselement als ebene Kopfplatte zur mechanischen Verbindung mit dem Trommelfell dient und das zweite Befestigungselement zur Anlage der Gehörknöchelchenprothese auf der Steigbügelfußplatte ausgebildet ist.

Eine weitere vorteilhafte Ausführungsform der erfindungsgemäßen Gehörknöchelchenprothese zeichnet sich dadurch aus, dass mindestens ein radial innerstes Puzzle-Teil vorgesehen ist, das den Ankoppelbereich enthält, und dass mindestens ein radial äußeres Puzzle-Teil das radial innerste Puzzle-Teil im zusammengeknöpften Zustand ringförmig umgibt. In der Regel wird man mehrere radiale Abfolgen von Puzzle-Ringen vorsehen. Auf diese Weise lässt sich die Fläche des ersten Befestigungselement wie beim Schälen einer Zwiebel je nach aktuellem Bedarf durch "Abknöpfen" einer entsprechenden Anzahl radial äußerer Puzzle-Ringe gezielt auf eine gewünschte Größe verkleinern. Übrigens kann durch "Anknöpfen" des zuletzt entfernten äußeren Puzzle-Rings ein zu klein geratenes Befestigungselement auch wieder vergrößert werden, was die Flexibilität des Operateurs nochmals erhöht.

Bevorzugt sind auch Ausführungsformen der Erfindung, bei denen mindestens ein Puzzle-Teil vorgesehen ist, das an mindestens ein anderes Puzzle-Teil am Außenrand des plattenförmigen ersten Befestigungselements in der Plattenebene von der Seite her angeknöpft werden kann. Dadurch eröffnen sich hinsichtlich der geometrischen Form des Befestigungselements enorm viele Gestaltungsmöglichkeiten. Insbesondere müssen jetzt nicht mehr unbedingt symmetrische Formen implantiert werden, wie sie bisher durch die vorhandenen Standard-Prothesen zwangsläufig vorgegeben waren. Vielmehr kann der Operateur ganz leicht und noch während der Operation eine handgefertigte, optimal angepasste Gehörknöchelchenprothese zielgenau "tailor-made" herstellen.

Bei vorteilhaften Weiterbildungen dieser Klasse von Ausführungsformen ist das seitlich anknöpfbare Puzzle-Teil als Appendix für den Hammer oder den Hammergriff der Gehörknöchelchenprothese geformt und ragt im zusammengeknöpften Zustand spitz vom Rand des plattenförmigen ersten Befestigungselements radial nach außen weg.

Diese Weiterbildungen lassen sich noch dadurch verbessern, dass das seitlich anknöpfbare Puzzle-Teil federnd im ersten Befestigungselement verankert ist, was insbesondere ein unbeabsichtigtes Abbrechen des sehr feinen Miniaturteils beim Anknöpfen in das Befestigungselement erschwert.

Die federnde Verankerung des Puzzle-Teils lässt sich beispielsweise mittels eines einfachen Klammerelements erreichen.

Bei weiteren vorteilhaften Varianten ist das seitlich anknöpfbare Puzzle-Teil einrastend und damit verliersicher im ersten Befestigungselement verankert.

Eine Klasse von Ausführungsformen der Erfindung zeichnet sich dadurch aus, dass das plattenförmige erste Befestigungselement im zusammen geknöpften Zustand der Puzzle-Teile Durchbrüche durch die Plattenebene aufweist. Damit lassen sich einerseits leicht auch die oben bereits erwähnten asymmetrischen Formgestaltungen in der Geometrie des Befestigungselements erreichen. Andererseits weist das plattenförmige Befestigungselement aufgrund der Durchbrüche eine hohe Flexibilität bezüglich lokaler Bewegungen von Teilen des Befestigungselements aus der Plattenebene heraus auf, was sich vor allem bei Ausgestaltungen des Befestigungselements als Kopfplatte zur Anlage am Trommelfell besonders vorteilhaft auswirkt.

Bei einer alternativen Klasse von Ausführungsformen bildet das plattenförmige erste Befestigungselement im zusammengeknöpften Zustand der Puzzle-Teile eine in sich geschlossene Fläche und weist eben gerade keine Durchbrüche durch die Plattenebene auf. Aufgrund dieser Ausgestaltung ist das Befestigungselement in sich besonders verwindungssteif.

Ganz besonders bevorzugt ist eine Ausführungsform der erfindungsgemäßen Gehörknöchelchenprothese, bei der die radial äußeren Abschnitte des plattenförmigen ersten Befestigungselements einen äußeren Ringbereich bilden. Diese Ausführungsform eignet sich insbesondere gut für das oben erwähnte schnelle sukzessive "Zwiebelschalenartige" Verkleinern der Fläche des ersten Befestigungselements radial von außen nach innen.

Eine Gruppe von Weiterbildungen dieser Ausführungsform zeichnet sich dadurch aus, dass der radial äußere Ringbereich ununterbrochen in sich selbst geschlossen verläuft. Auf diese Weise kann die Fläche des Befestigungselements durch Abknöpfen ganzer Ringbereiche radial von außen nach innen symmetrisch verkleinert werden. Außerdem kann dadurch bei post-operativen Retraktionen die Ausbildung von gegen das Trommelfell gerichteten, gefährlichen Spitzen vermieden werden.

Alternativ dazu ist bei einer anderen Gruppe von Weiterbildungen vorgesehen, dass der radial äußere Ringbereich mindestens eine, vorzugsweise mehrere Unterbrechungen aufweist. Hiermit lassen sich insbesondere asymmetrische Formgestaltungen leicht bewerkstelligen.

Der radial äußere Ringbereich kann bei Weiterbildungen der oben beschriebenen Ausführungsform eine ovale oder kreisrunde Form aufweisen, was aus dem Stand der Technik an sich bekannt ist und in der Regel die Standard-Variante für die erfindungsgemäße Gehörknöchelchenprothese bilden wird.

Zur Erleichterung des operativen Einsetzens der erfindungsgemäßen Gehörknöchelchenprothese kann bei einer speziellen Variante der radial äußere Ringbereich eine einseitige Einbuchtung zur Aufnahme des Hammergriffs aufweisen.

Eine weitere Variante sieht vor, dass der radial äußere Ringbereich des plattenförmigen ersten Befestigungselements in der Plattenebene eine radial nach außen verlaufende Ausbuchtung aufweist, die in Strukturen der Gehörknöchelchenkette eingreifen kann.

Möglich ist aber auch eine Variante, bei der der radial äußere Ringbereich eine Schlangenlinienförmige Außenkontur aufweist, die sich in speziellen geometrischen Mittetohrsituationen, wie sie beim Patienten in der Praxis oftmals vorgefunden werden können, als günstig erweist.

Eine weitere Ausführungsform der erfindungsgemäßen Gehörknöchelchenprothese zeichnet sich dadurch aus, dass die Stegelemente zwischen dem radial inneren Ankoppelbereich und den radial äußeren Abschnitten des plattenförmigen ersten Befestigungselements ununterbrochen verlaufen, um die oben erwähnte gewünschte Flexibilität zu erhöhen.

Alternativ können aber auch zumindest einige Stegelemente eine Unterbrechung zwischen dem radial inneren Ankoppelbereich und den radial äußeren Abschnitten des plattenförmigen ersten Befestigungselements aufweisen, was die Flexibilität hinsichtlich Verwindungen des Befestigungselements in seiner Plattenebene erhöht.

Besonders bevorzugt sind Ausführungsformen der Erfindung, bei denen die Stegelemente des plattenförmigen ersten Befestigungselements in der Plattenebene nicht geradlinig, sondern auf mehrfach gekrümmten Kurven verlaufen, wodurch sich die erwünschte Wirkung einer lokalen Flexibilität einer Trommelfell-Kopfplatte und eines lediglich lokal begrenzten Ausweichens bei kleineren Medialbewegungen des Trommelfells noch verstärkt. Außerdem kann dadurch eine Kopfplatte einer eventuellen post-operativen Veränderung des Trommelfells leichter folgen.

Bei weiteren vorteilhaften Ausführungsformen der erfindungsgemäßen Gehörknöchelchenprothese ist jedes Stegelement mit mindestens zwei anderen Stegelementen verbunden, so dass eine Art flexibles Netzwerk aus Stegelementen entsteht.

Besonders günstig ist es, wenn die Stegelemente eine maximale Breite b und der radial äußere Ringbereich eine maximale Breite B aufweisen, wobei gilt: 2b < B.

Um die gewünschte Flexibilität zu erreichen, sollte bei einer maximalen Breite b der Stegelemente und einem minimalen Durchmesser D des plattenförmigen ersten Befestigungselements einschließlich des Ringbereiches gelten: b ≤ 0,05D, vorzugsweise b ≈ 0,03D. Beim Stand der Technik, etwa bei den in der US 2004/0162614 A1 beschriebenen Gehörknöchelchenprothesen, liegt das Verhältnis b/D mindestens bei 0,1 oder darüber.

Weiter ist es günstig, wenn das plattenförmige erste Befestigungselement der erfindungsgemäßen Gehörknöchelchenprothese eine Dicke, insbesondere eine Blechdicke t zwischen 0,01mm und 0,5mm, vorzugsweise zwischen 0,1mm und 0,25mm, sowie einen minimalen Durchmesser D zwischen 1,5mm und 8mm, vorzugsweise zwischen 2mm und 5mm, und die Stegelemente eine maximale Breite b zwischen 0,01mm und 0,3mm, vorzugsweise zwischen 0,05mm und 0,2mm aufweisen.

In der Regel wird bei der erfindungsgemäßen GehörknöchelchenProthese das Verbindungselement zwischen den Befestigungselementen als länglicher Schaft ausgeführt, wie dies an sich aus dem Stand der Technik wohlbekannt ist.

Um die oben erörterte Flexibilität bzw. Variabilität der Prothese - wie sie an sich in der EP 1 181 907 B1 beschrieben ist - zu erhöhen, kann bei einer besonders bevorzugten Weiterbildung dieser Ausführungsform am oder im länglichen Schaft mindestens ein Kugelgelenk vorgesehen sein. Vorteilhaft im Hinblick auf eine besonders hohe postoperative Beweglichkeit der Prothese sind Varianten, bei denen der längliche Schaft eine Vielzahl von aneinander angrenzenden weiteren Drehelementen, vorzugsweise eine Kugelgelenkkette, umfasst.

Alternativ kann der Schaft bei besonders einfachen und preisgünstig herstellbaren Ausführungsformen der erfindungsgemäßen Prothese aber auch einstückig durchgehend, insbesondere starr ausgeführt sein.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. In vielen in der Praxis eingesetzten Ausführungsformen der Erfindung wird das erste Befestigungselement eine zur Anlage am Trommelfell ausgebildete Kopfplatte umfassen. Bei vielen weiteren Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke, als Stempel oder als Clip zur mechanischen Verbindung mit einem weiteren Glied der Gehörknöchelchenkette ausgebildet ist.

Bei Weiterbildungen dieser Ausführungsformen ist die Prothese über das als Kopfplatte ausgebildete erste Befestigungselement einerseits am Trommelfell und über das zweite Befestigungselement andererseits am Amboss oder am Steigbügel befestigt.

Alternative Ausgestaltungen können vorsehen, dass die Gehörknöchelchenprothese mittels Perforation der Steigbügelfußplatte (=Stapedektomie bzw. Stapedotomie) und/oder mittels Eröffnung der menschlichen Hörschnecke (=Cochleotomie) an dem der Trommelfellseite entgegen gesetzten anderen Ende direkt an das Innenohr angekoppelt ist, insbesondere über einen Kolben.

Möglich sind Ausführungsformen der Erfindung, bei denen die Prothese oder Teile davon aus biokompatiblen Kunststoffen, insbesondere Silikon, Polytetrafluorethylen (PTFE) oder Polyetheretherketon (PEEK), und/oder aus Faserverbundwerkstoffen, insbesondere Kohlefasern hergestellt sind. Mit diesen Materialien können postoperative Abstoßungsreaktionen in den meisten Fällen verhindert werden.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgebildetes Befestigungselement sollte bei der erfindungsgemäßen Gehörknöchelchenprothese grundsätzlich eine wachstumsfördernde Beschichtung, ein direkt ins Innenohr führendes, etwa in Form eines Kolbens ausgebildetes Befestigungselement hingegen eine wachstumshemmende Beschichtung aufweisen.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein mechanisches Tuning der Schallfortpflanzungseigenschaften mittels einer individuellen ausgestalteten Gehörknöchelchenprothese erreichen.

Ein solcher Tuning-Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung schließlich zeichnet sich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Gehörknöchelchenprothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Die Figuren der Zeichnung sind in 3er-Gruppen unterteilt, wobei die jeweiligen Einzelfiguren einer Gruppe voneinander durch eine Nummerierung mit a, b oder c unterschieden sind. Die "a"-Figuren enthalten jeweils eine schematische räumliche Darstellung einer Ausführungsform der erfindungsgemäßen Gehörknöchelchenprothese, die "b"-Figuren das der entsprechenden "a"-Figur zugehörige, erfindungsgemäß ausgestaltete erste Befestigungselement im verbundenen Zustand der Puzzle-Teile und die "c"-Figuren das erste Befestigungselement der entsprechenden "b"-Figur im auseinander geknöpften Zustand der Puzzle-Teile. Im Übrigen sind in der Zeichnung Elemente mit gleichem Aufbau und/oder gleicher Funktion mit der derselben Bezugsziffer gekennzeichnet.

### Im Einzelnen zeigen:

- Fign. 1a-c: eine Ausführungsform der erfindungsgemäßen Gehörknöchelchenprothese mit einem als Trommelfell-Kopfplatte gestalteten, ringförmig aufgebauten ersten Befestigungs-element und einem als geschlitzte Glocke ausgeführten zweiten Befestigungselement am anderen Ende des Verbindungselements;
- Fign. 2a-c: eine Ausführungsform mit zwei plattenförmig aufgebauten Befestigungselementen;
- Fign. 3a-c: eine Ausführungsform mit einem seitlich anknöpfbaren, als Appendix für den Hammer oder den Hammergriff geformten Puzzle-Teil am ersten Befestigungselement sowie einem klammerförmigen zweiten Befestigungselement;
- Fign. 4a-c: eine Ausführungsform mit seitlich anknöpfbarem Puzzle-Teil und radial nach außen verlaufender Ausbuchtung des ersten Befestigungselements nach dem Abknöpfen des anknöpfbaren Puzzle-Teils;
- Fign. 5a-c: eine Ausführungsform mit einem federnd im ersten Befestigungselement verankerten, seitlich anknöpfbaren Puzzle-Teil;
- Fign. 6a-c: eine Ausführungsform mit einem mittels eines Klammerelements im ersten Befestigungselement verankerten seitlich anknöpfbaren Puzzle-Teil;
- Fign. 7a-c: eine Ausführungsform mit einem einrastend im ersten Befestigungselement verankerten, seitlich anknöpfbaren Puzzle-Teil;
- Fign. 8a-c: eine Ausführungsform, bei der das plattenförmige erste Befestigungselement im zusammengeknöpften Zustand der Puzzle-Teile eine in sich geschlossene Fläche bildet und keine Durchbrüche durch die Plattenebene aufweist; und
- Fign. 9a-c: eine Ausführungsform mit einem als Trommelfell-Kopfplatte gestalteten, ringförmig aufgebauten ersten Befestigungs-element, einem Kugelgelenk im Verbindungselement und einem kolbenförmigen zweiten Befestigungselement.

Die erfindungsgemäßen **Gehörknöchelchenprothesen 10; 20; 30; 40; 50; 60; 70; 80; 90,** weisen jeweils an einem Ende ein plattenförmiges **erstes Befestigungselement 11; 21; 31; 41; 51; 61; 71; 81; 91** auf, welches in Form einer Kopfplatte zur Anlage am Trommelfell oder als Fußplatte zur Anlage an der Steigbügelfußplatte ausgebildet ist. Am anderen Ende der Gehörknöchelchenprothesen 10; 20; 30; 40; 50; 60; 70; 80; 90 sitzt jeweils ein **zweites Befestigungselement 12; 22; 32; 92** zur mechanischen Verbindung der Prothese mit einem Glied oder Teilen eines Gliedes der Gehörknöchelchen-Kette oder direkt mit dem Innenohr. Dazwischen ist ein die beiden Befestigungselemente Schall leitend miteinander verbindendes **Verbindungselement 13; 23; 93** angeordnet, welches bei den gezeigten Ausführungsformen in Form eines ein- oder mehrteiligen, kurzen oder längeren Schaftes ausgeführt ist.

Das plattenförmige erste Befestigungselement 11; 21; 31; 41; 51; 61; 71; 81; 91 weist jeweils einen radial inneren, insbesondere um seinen Flächenschwerpunkt zentral angeordneten **Ankoppelbereich 14; 24; 34; 44; 54; 64; 74; 84; 94** zum mechanischen Ankoppeln des ersten Befestigungselements an das Verbindungselement 13; 23; 93 sowie mehrere **Stegelemente 15, 15', 15"; 25, 25', 25"; 35, 35', 35", 35"'; 45, 45'; 55, 55', 55"; 65, 65'; 75, 75', 75"; 85, 85'; 95, 95'** zur radialen Verbindung des radial inneren Ankoppelbereichs mit radial **äußeren Abschnitten 16, 16'; 26, 26'; 36, 36'; 46, 46'; 56, 56'; 66, 66'; 76, 76'; 86, 86'; 96, 96'** des ersten Befestigungselements 11; 21; 31; 41; 51; 61; 71; 81; 91 auf.

Der Ankoppelbereich 14; 24; 34; 44; 54; 64; 74; 84; 94 und/oder die Stegelemente 15, 15', 15"; 25, 25', 25"; 35, 35', 35", 35"'; 45, 45'; 55, 55', 55"; 65, 65'; 75, 75', 75"; 85, 85'; 95, 95' und/oder die radial äußeren Abschnitte 16, 16'; 26, 26'; 36, 36'; 46, 46'; 56, 56'; 66, 66'; 76, 76'; 86, 86'; 96, 96' sind geometrisch so gestaltet, dass sie zusammen Teile eines Puzzles bilden, aus denen das plattenförmige erste Befestigungselement 11; 21; 31; 41; 51; 61; 71; 81; 91 vollständig zusammengesetzt werden kann, wobei die einzelnen Puzzle-Teile knöpfbar aneinander gefügt oder auseinander genommen werden können, und wobei das erste Befestigungselement 11; 21; 31; 41; 51; 61; 71; 81; 91 im zusammengeknöpften Zustand der Puzzle-Teile in sich mechanisch stabil ist.

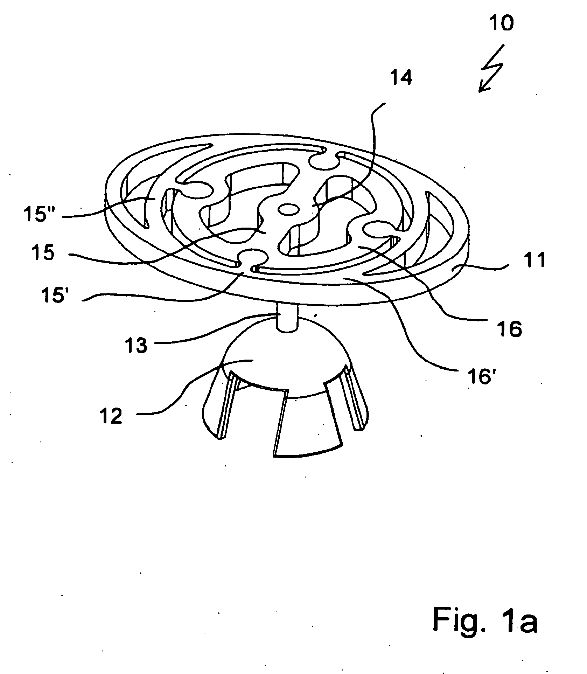

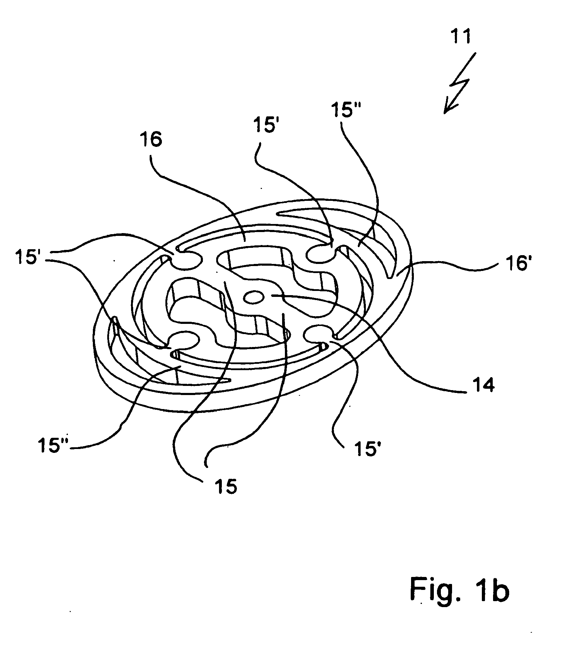

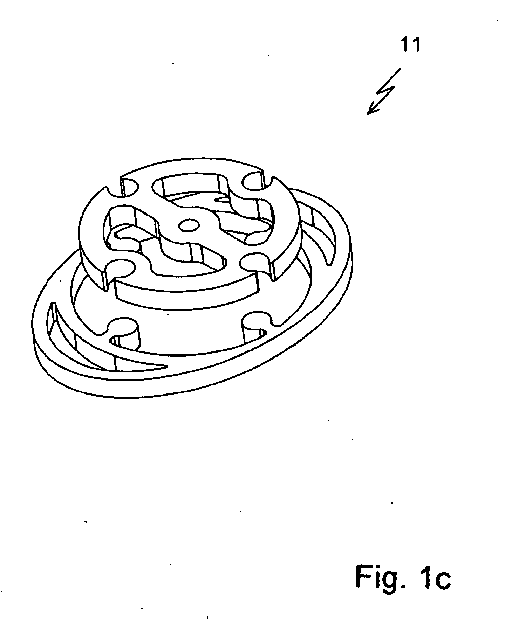

Die in den Figuren 1a-c dargestellte Ausführungsform weist als erstes Befestigungselement 11 eine Kopfplatte und als Verbindungselement 13 einen einstückig durchgehenden, starren kurzen Schaft sowie ein zweites Befestigungselement 12 mit einer glockenartigen Gestaltung auf, welches zur Befestigung der Gehörknöchelchenprothese 10 an einem Glied der Gehörknöchelchenkette dient, beispielsweise am Amboss oder am Steigbügel. Das erste Befestigungselement 11 weist Durchbrüche durch die Plattenebene zur Erhöhung der Flexibilität der Kopfplatte bei Verwindungen in und aus der Plattenebene auf und umfasst ein radial inneres Puzzle-Teil 16, das den Ankoppelbereich 14 enthält. Ein weiteres, radial äußeres Puzzle-Teil 16' umgibt das radial innere Puzzle-Teil 16 im zusammengeknöpften Zustand ringförmig, wobei der radial äußere Ringbereich eine kreisrunde Form hat und ununterbrochen in sich selbst geschlossen verläuft.

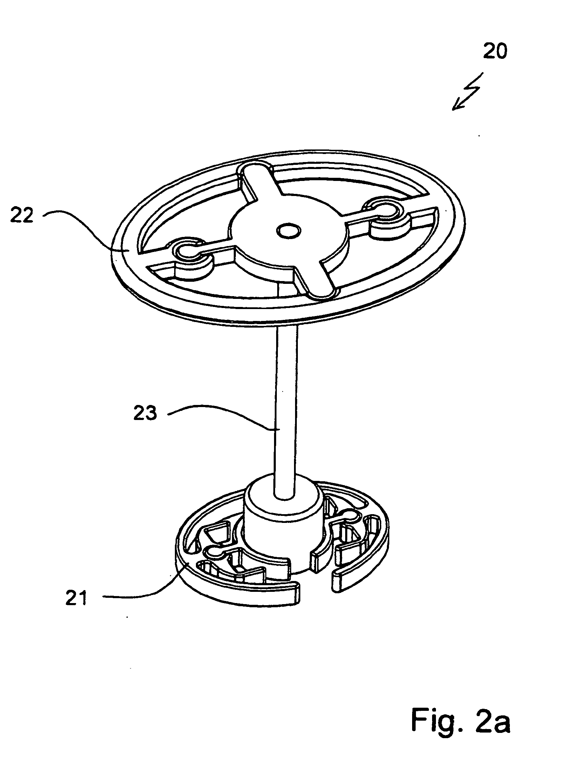

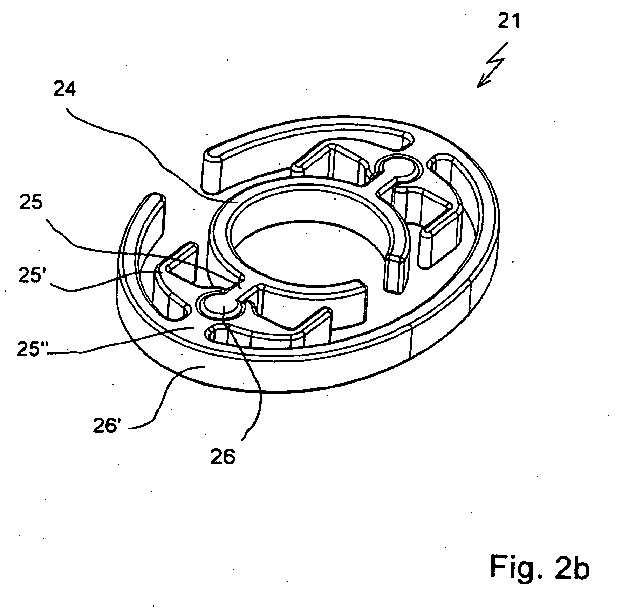

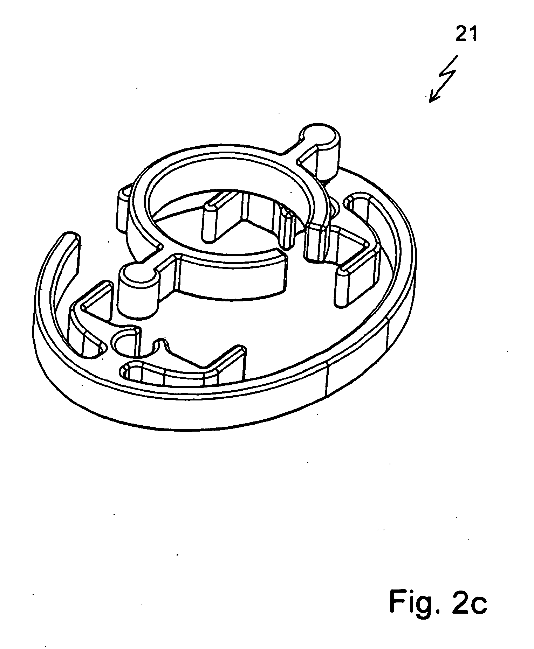

Bei der Ausführungsform nach den Figuren 2a-c sind beide Befestigungselemente 21, 22 plattenförmig gestaltet und erfindungsgemäß aus Puzzle-Teilen aufgebaut, wobei das erste Befestigungselement 21 zur Anlage der Gehörknöchelchenprothese 20 auf der Steigbügelfußplatte ausgebildet ist, während das zweite Befestigungselement 22 als ebene Kopfplatte zur mechanischen Verbindung mit dem Trommelfell dient. Da es sich hier um eine Totalprothese handelt, ist das schaftförmige Verbindungselement 23 etwas länger als der kurze Schaft 13 in Fig. 1a. Auch beim ersten Befestigungselement 21 wird ein radial inneres Puzzle-Teil 26, das den Ankoppelbereich 24 enthält, von einem weiteren, radial äußeren Puzzle-Teil 26' ringförmig umgeben, wobei jedoch sowohl der radial innere als auch der radial äußere Ringbereich jeweils eine Unterbrechung aufweisen, die die Flexibilität des plattenförmigen ersten Befestigungselements 21 bei Verwindungen in und aus der Plattenebene erhöht.

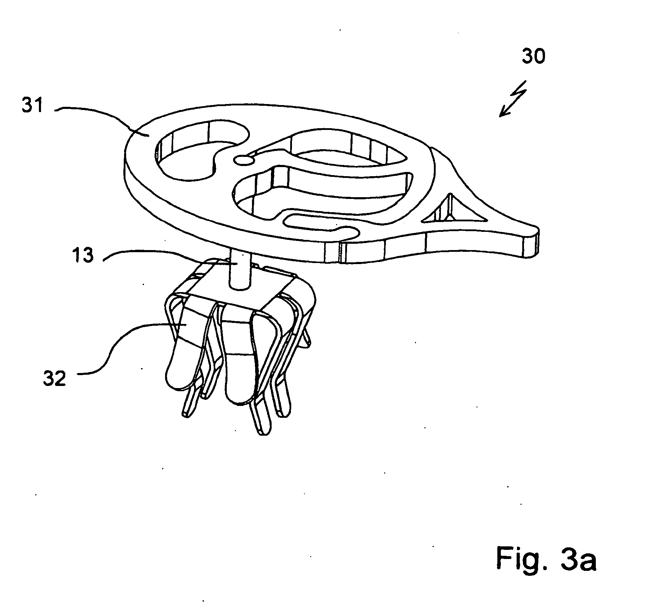

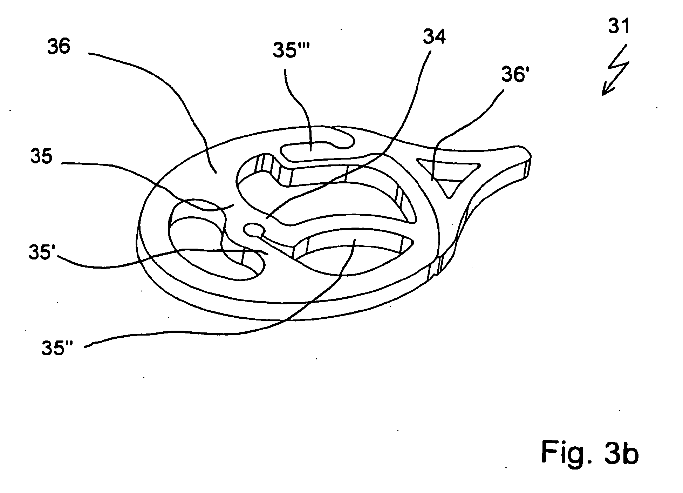

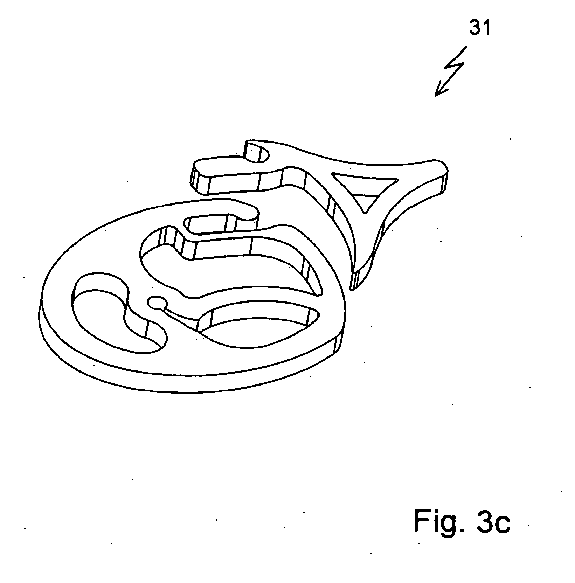

Die Ausführungsformen der Figuren 3a bis 9c zeichnen sich dadurch aus, dass jeweils mindestens ein Puzzle-Teil 36'; 46'; 56'; 66'; 76'; 86'; 96' vorgesehen ist, das an mindestens ein anderes Puzzle-Teil 36; 46; 56; 66; 76; 86; 96 am Außenrand des plattenförmigen ersten Befestigungselements 31; 41; 51; 61; 71; 81; 91 in der Plattenebene von der Seite her angeknöpft werden kann. Bei den Ausführungsformen der Figuren 3a bis 7c ist dieses seitlich anknöpfbare Puzzle-Teil 36'; 46'; 56'; 66'; 76' jeweils als Appendix für den Hammer oder den Hammergriff der Gehörknöchelchenprothese 30; 40; 50; 60; 70 geformt und ragt im zusammengeknöpften Zustand spitz vom Rand des plattenförmigen ersten Befestigungselements 31; 41; 51; 61; 71 radial nach außen weg.

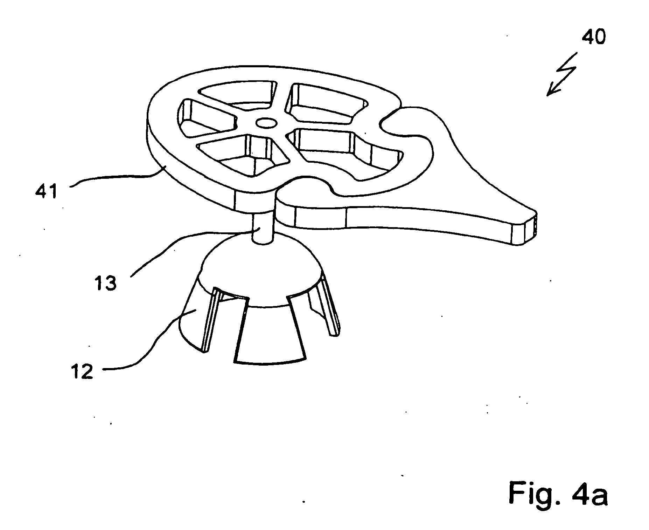

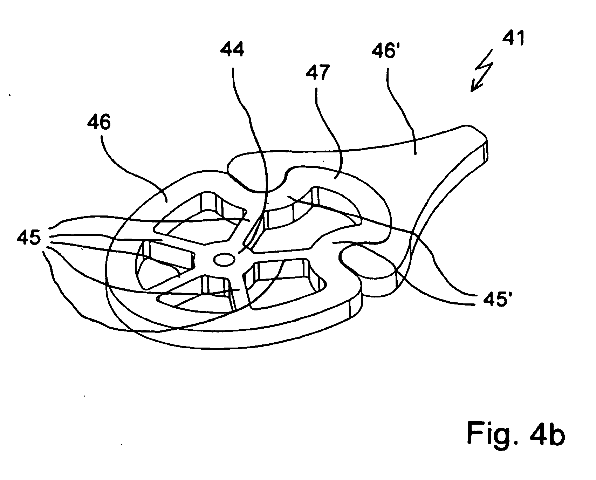

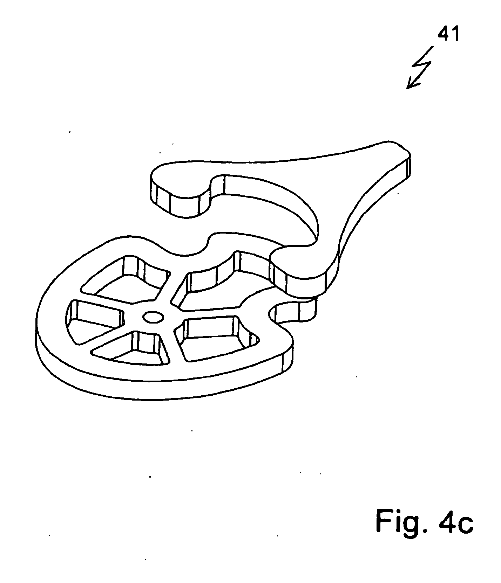

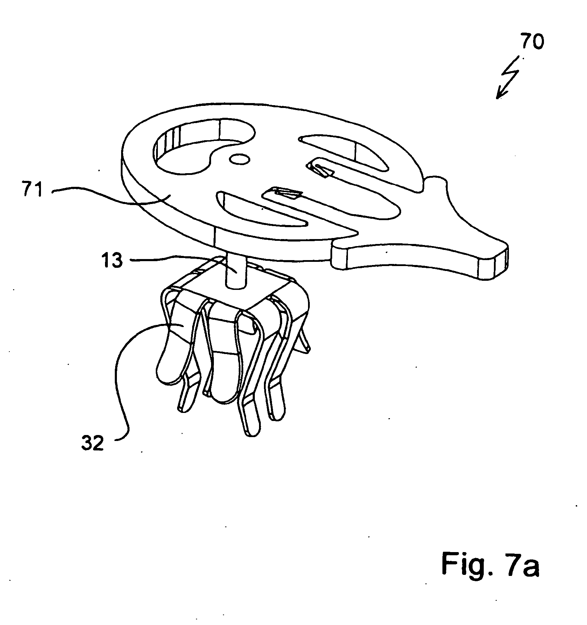

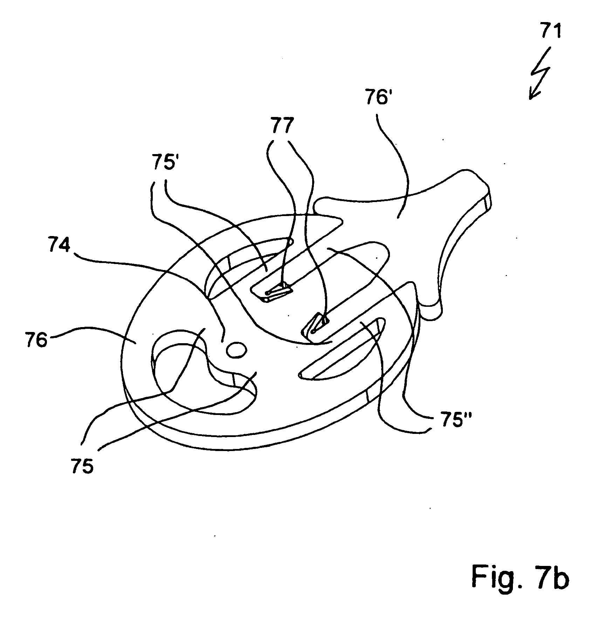

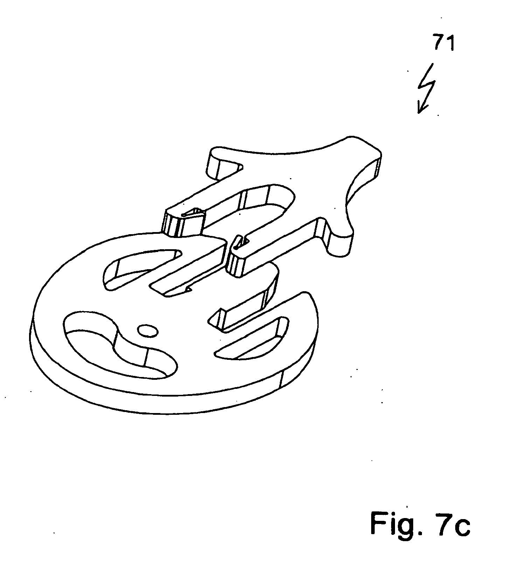

Die zweiten Befestigungselemente 32; 72 bei den Gehörknöchelchenprothesen 30; 70 nach den Figuren 3a; 7a sind jeweils als Klammer mit mehreren Zungen ausgeführt, während sie bei den Ausführungsformen nach den Figuren 4a bis 6a jeweils eine geschlitzte Glockenform wie das zweite Befestigungselement 12 in Fig. 1a aufweisen.

Das plattenförmige erste Befestigungselement 41 der Ausführungsform nach den Figuren 4a-c zeichnet sich dadurch aus, dass sein erstes Puzzle-Teil 46 im radial äußeren Ringbereich eine in der Plattenebene radial nach außen verlaufende Ausbuchtung 47 aufweist, die auch schon ohne das zweite, seitlich anknöpfbare Puzzle-Teil 46' als Appendix für den Hammer oder den Hammergriff der Gehörknöchelchenprothese 40 dienen kann.

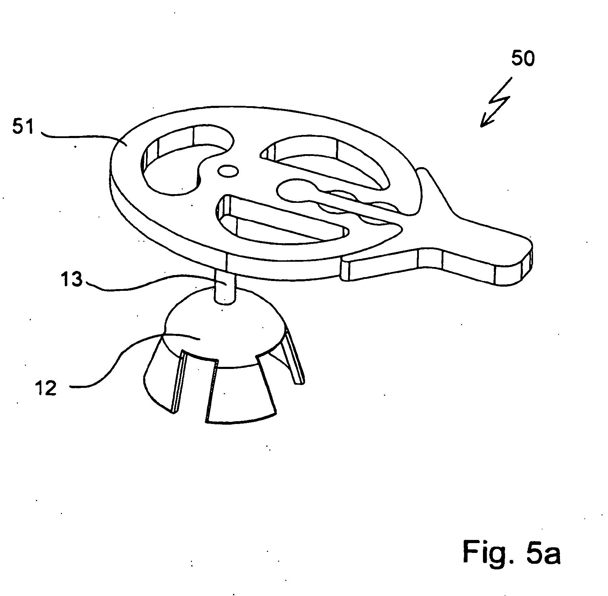

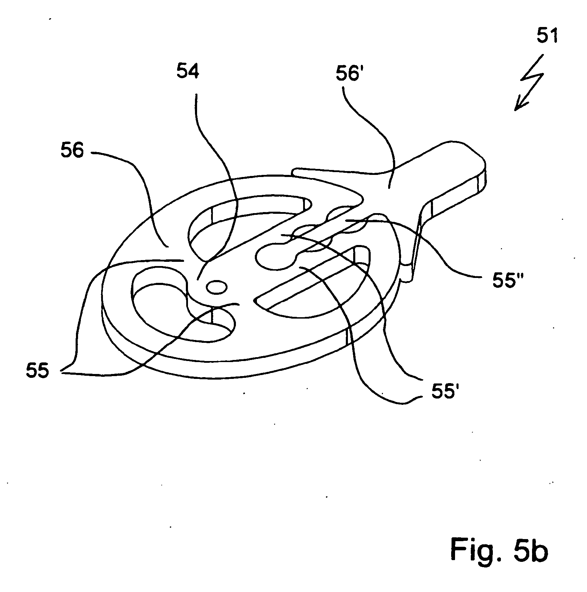

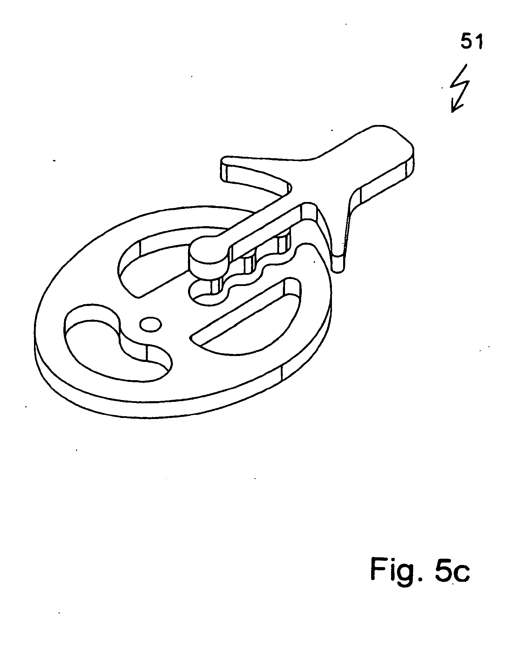

Bei den ersten Befestigungselementen 51; 61; 71 der Gehörknöchelchenprothesen 50; 60; 70 gemäß den Figuren 5a bis 7c ist jeweils das seitlich anknöpfbare Puzzle-Teil 56'; 66'; 76' federnd im ersten Befestigungselement 51; 61; 71 verankert.

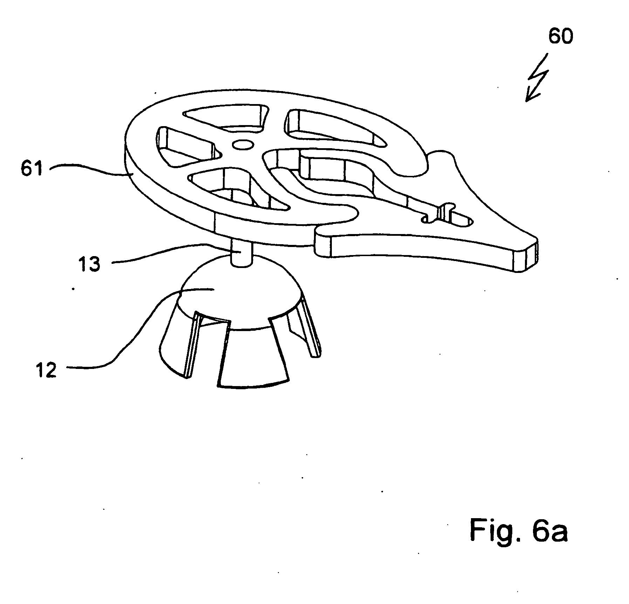

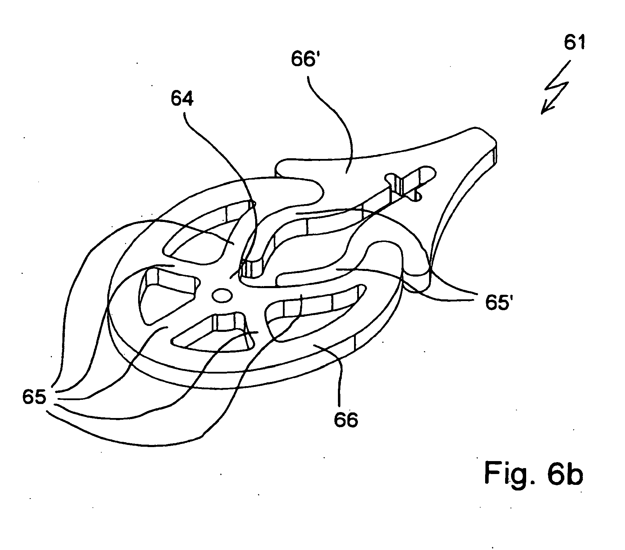

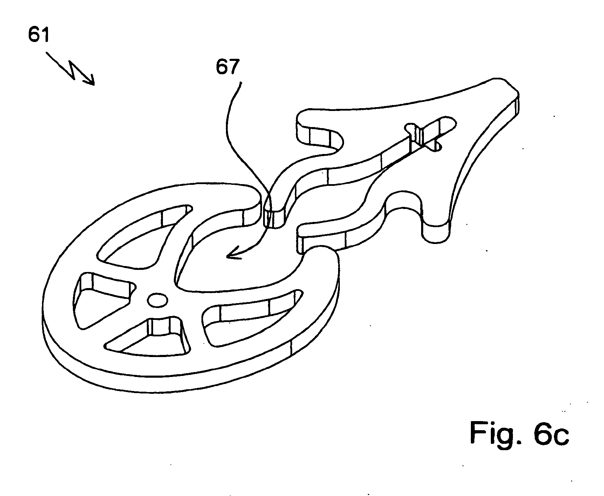

Die anknöpfbaren Puzzle-Teile 66'; 76' der Ausführungsformen nach den Figuren 6a bis 7c können jeweils mittels eines als Klammerelement ausgebildeten Paares von Stegelementen 65' bzw. 75" im jeweiligen ersten Puzzle-Teil 66 bzw. 76 des ersten Befestigungselements 61; 71 seitlich verankert werden.

Der radial äußere Ringbereich des ersten Befestigungselements 61 in der Ausführungsform nach den Figuren 6a-c weist eine einseitige **Einbuchtung 67** auf, die zur Aufnahme des Hammergriffs dienen kann, wenn das anknöpfbare Puzzle-Teil 66' nicht verwendet wird.

Bei der Gehörknöchelchenprothese 70 nach den Figuren 7a-c wird das seitlich anknöpfbare Puzzle-Teil 76' mittels an den Enden der beiden Stegelemente 75" vorgesehener **Widerhaken 77** einrastend und damit verliersicher im ersten Befestigungselement 71 verankert.

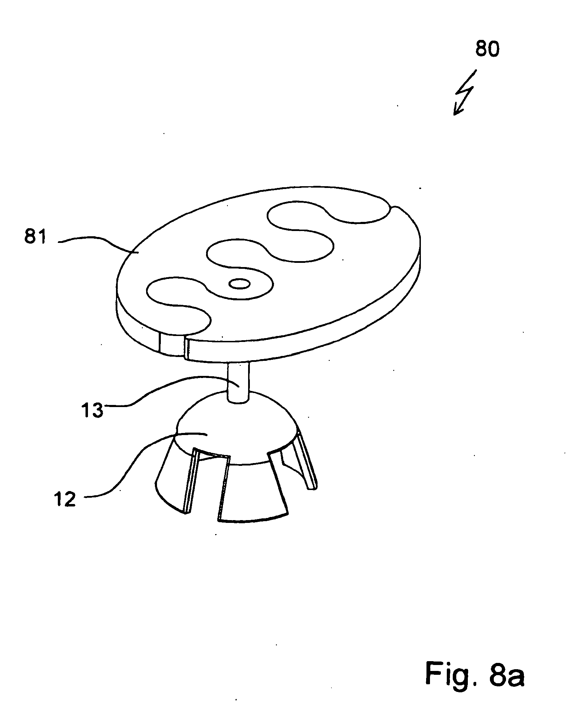

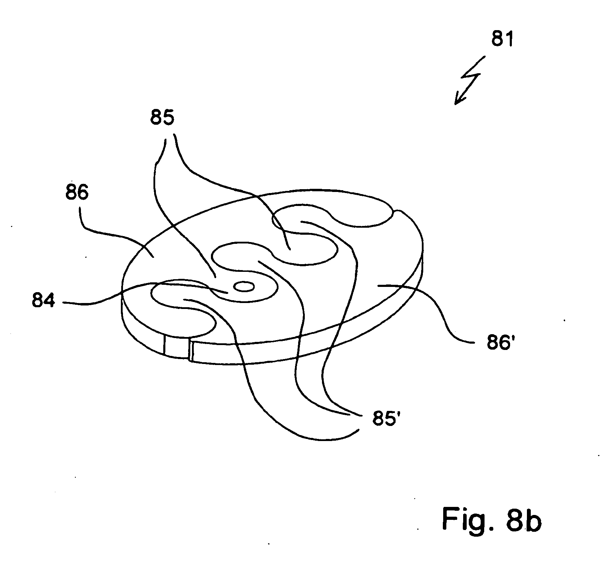

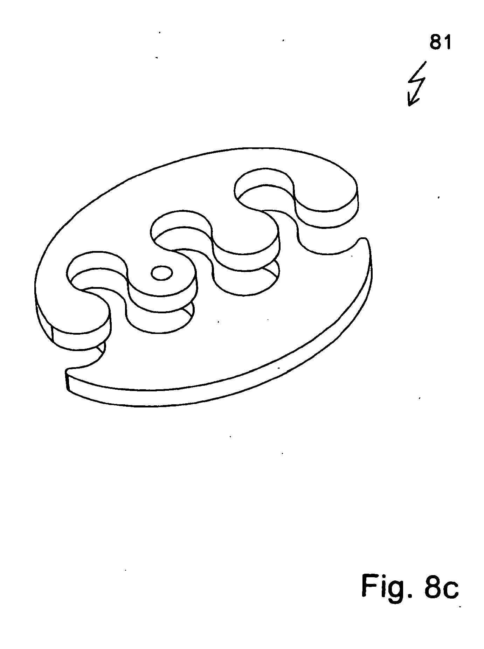

In den Figuren 8a-c ist eine Ausführungsform der Erfindung dargestellt, bei der das plattenförmige erste Befestigungselement 81 im zusammengeknöpften Zustand der Puzzle-Teile 86, 86' eine in sich geschlossene Fläche bildet und keine Durchbrüche durch die Plattenebene aufweist, wodurch die Platte relativ verwindungssteif ist.

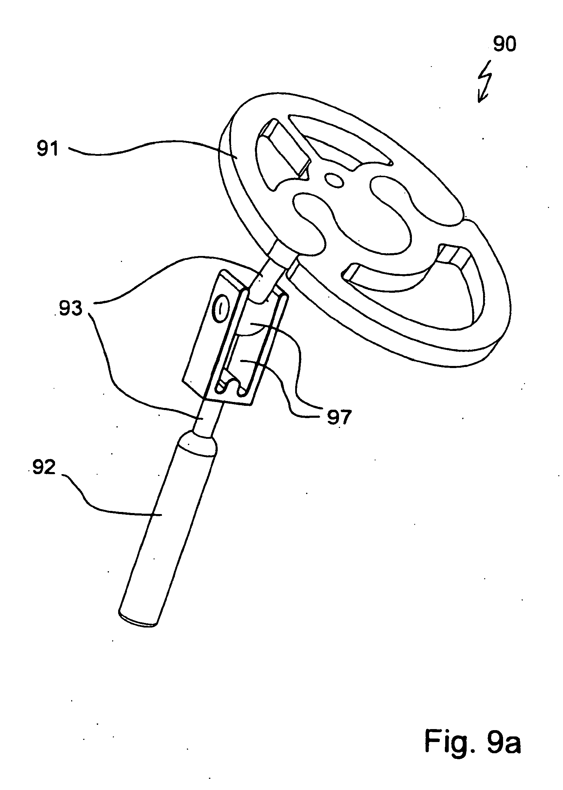

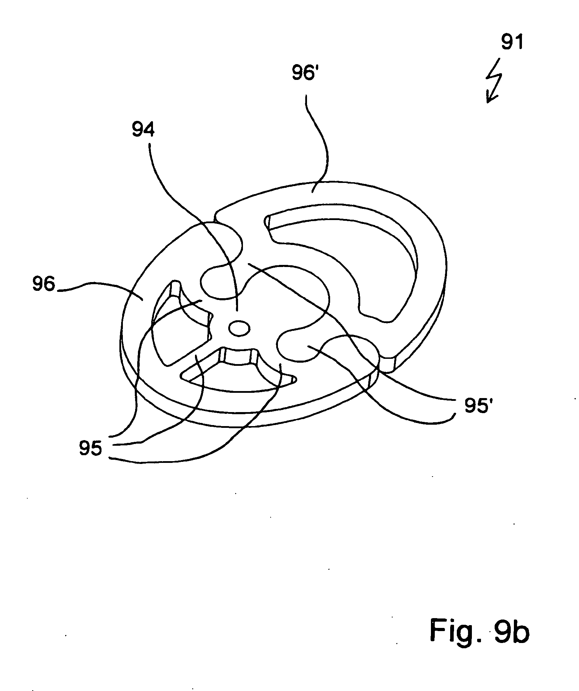

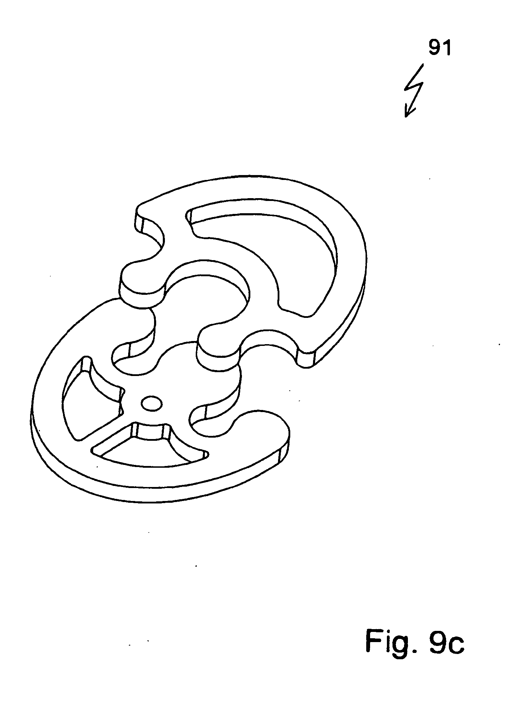

Die Figuren 9a-c schließlich zeigen eine Ausführungsform mit einem wiederum als Kopfplatte zur Anlage am Trommelfell ausgebildeten ersten Befestigungselement 91. Das zweite Befestigungselement 92 an dem der Kopfplatte entgegen gesetzten Ende der in Fig. 9a dargestellten Gehörknöchelchenprothese 90 ist im vorliegenden Ausführungsbeispiel als Kolben zur direkten Ankopplung der Gehörknöchelchenprothese 90 an das Innenohr ausgebildet. Das schaftförmige Verbindungselement 93 ist hier zweigeteilt aufgebaut und weist zwischen seinen beiden Teilen ein **Kugelgelenk 97** auf, um die mechanische Flexibilität des Schaftes zu erhöhen.

Die Massenverteilung der einzelnen Teile der erfindungsgemäßen Gehörknöchelchenprothese 10; 20; 30; 40; 50; 60; 70; 80; 90 kann in Abhängigkeit von einem gewünschten, vorgebbaren Frequenzgang der Schallleitung im Mittelohr berechnet sein, um ein individuelles Tuning der Schallleitungs-Eigenschaften zu ermöglichen.

Bei in der Zeichnung nicht eigens dargestellten weiteren Ausführungsformen der erfindungsgemäßen Gehörknöchelchenprothese können die zentralen Ankoppelbereiche und/oder die Stegelemente und/oder die radial äußeren Abschnitte auch andere Geometrien besitzen, um die gewünschte Flächenvariabilität des jeweiligen ersten Befestigungselements zu erzielen. So können etwa zumindest einige Stegelemente eine Unterbrechung zwischen dem zentralen Ankoppelbereich und den radial äußeren Abschnitten aufweisen. Auch können die radial äußeren Abschnitte einfach oder mehrfach unterbrochen gestaltet sein, eine Schlangenlinienförmige Außenkontur und/oder eine einseitige Einbuchtung zur Aufnahme des Hammergriffs aufweisen.

## Patentansprüche

1. Gehörknöchelchenprothese (10; 20; 30; 40; 50; 60; 70; 80; 90), die mindestens ein Glied oder Teile eines Gliedes der Gehörknöchelchenkette ersetzt oder überbrückt, wobei die Gehörknöchelchenprothese (10; 20; 30; 40; 50; 60; 70; 80; 90) an ihrem einen Ende ein im Wesentlichen plattenförmiges erstes Befestigungselement (11; 21; 31; 41; 51; 61; 71; 81; 91) zur Anlage am Trommelfell oder an der Steigbügelfußplatte und an ihrem anderen Ende ein zweites Befestigungselement (12; 22; 32; 92) zur mechanischen Verbindung mit einem Glied oder Teilen eines Gliedes der Gehörknöchelchenkette oder mit dem Innenohr sowie ein die beiden Befestigungselemente (11,12; 21,22; 31,32; 41,12; 51,12; 61,12; 71,32; 81,12; 91,92) Schall leitend miteinander verbindendes Verbindungselement (13; 23; 93) umfasst, und wobei das plattenförmige erste Befestigungselement (11; 21; 31; 41; 51; 61; 71; 81; 91) einen radial inneren Ankoppelbereich (14; 24; 34; 44; 54; 64; 74; 84; 94) zum mechanischen Ankoppeln des ersten Befestigungselements (11; 21; 31; 41; 51; 61; 71; 81; 91) an das Verbindungselement (13; 23; 93) sowie mehrere Stegelemente (15,15',15"; 25,25',25"; 35,35',35",35"'; 45,45'; 55,55',55"; 65,65'; 75,75',75"; 85,85'; 95,95') zur radialen Verbindung des radial inneren Ankoppelbereichs (14; 24; 34; 44; 54; 64; 74; 84; 94) mit radial äußeren Abschnitten (16,16'; 26,26'; 36,36'; 46,46'; 56,56'; 66,66'; 76,76'; 86,86'; 96,96') des ersten Befestigungselements (11; 21; 31; 41; 51; 61; 71; 81; 91) aufweist,
**dadurch gekennzeichnet,**
**dass** der Ankoppelbereich (14; 24; 34; 44; 54; 64; 74; 84; 94) und/oder die Stegelemente (15,15',15"; 25,25',25"; 35,35',35", 35"'; 45,45'; 55,55',55"; 65,65'; 75,75',75"; 85,85'; 95,95') und/oder die radial äußeren Abschnitte (16,16'; 26,26'; 36,36'; 46,46'; 56,56'; 66,66'; 76,76'; 86,86'; 96,96') geometrisch so gestaltet sind, dass sie zusammen Teile eines Puzzles bilden, aus denen das plattenförmige erste Befestigungselement (11; 21; 31; 41; 51; 61; 71; 81; 91) vollständig zusammengesetzt werden kann, wobei die einzelnen Puzzle-Teile knöpfbar aneinander gefügt oder auseinander genommen werden können, und wobei das erste Befestigungselement (11; 21; 31; 41; 51; 61; 71; 81; 91) im zusammengeknöpften Zustand der Puzzle-Teile in sich mechanisch stabil ist.

2. Gehörknöchelchenprothese nach Anspruch 1, **dadurch gekennzeichnet, dass** beide Befestigungselemente (21,22) plattenförmig gestaltet und aus Puzzle-Teilen aufgebaut sind, wobei das erste Befestigungselement (21) als ebene Kopfplatte zur mechanischen Verbindung mit dem Trommelfell, das zweite Befestigungselement (22) zur Anlage der Gehörknöchelchenprothese (20) auf der Steigbügelfußplatte ausgebildet ist.

3. Gehörknöchelchenprothese nach einem der vorhergehenden Ansprüche, **dadurch gekennzeichnet, dass** mindestens ein radial inneres Puzzle-Teil (16; 26) vorgesehen ist, das den Ankoppelbereich (14; 24) enthält, und dass mindestens ein radial äußeres Puzzle-Teil (16'; 26') das radial innere Puzzle-Teil (16; 26) im zusammengeknöpften Zustand ringförmig umgibt.

4. Gehörknöchelchenprothese nach einem der vorhergehenden Ansprüche, **dadurch gekennzeichnet, dass** mindestens ein Puzzle-Teil (36'; 46'; 56'; 66'; 76'; 86'; 96') vorgesehen ist, das an mindestens ein anderes Puzzle-Teil (36; 46; 56; 66; 76; 86; 96) am Außenrand des plattenförmigen ersten Befestigungselements (31; 41; 51; 61; 71; 81; 91) in der Plattenebene von der Seite her angeknöpft werden kann.

5. Gehörknöchelchenprothese nach Anspruch 4, **dadurch gekennzeichnet, dass** das seitlich anknöpfbare Puzzle-Teil (36'; 46'; 56'; 66'; 76') als Appendix für den Hammer oder den Hammergriff der Gehörknöchelchenprothese (30; 40; 50; 60; 70) geformt ist und im zusammengeknöpften Zustand spitz vom Rand des plattenförmigen ersten Befestigungselements (31; 41; 51; 61; 71) radial nach außen wegragt.

6. Gehörknöchelchenprothese nach Anspruch 4 oder 5, **dadurch gekennzeichnet, dass** das seitlich anknöpfbare Puzzle-Teil (56'; 66'; 76') federnd im ersten Befestigungselement (51; 61; 71) verankert ist.

7. Gehörknöchelchenprothese nach Anspruch 6, **dadurch gekennzeichnet, dass** das seitlich anknöpfbare Puzzle-Teil (66'; 76') mittels eines Klammerelements im ersten Befestigungselement (61; 71) verankert ist.

8. Gehörknöchelchenprothese nach einem der Ansprüche 4 bis 7, **dadurch gekennzeichnet, dass** das seitlich anknöpfbare Puzzle-Teil (76') einrastend im ersten Befestigungselement (71) verankert ist, insbesondere mittels Widerhaken (77).

9. Gehörknöchelchenprothese nach einem der Ansprüche 1 bis 8, **dadurch gekennzeichnet, dass** das plattenförmige erste Befestigungselement (11; 21; 31; 41; 51; 61; 71; 91) im zusammengeknöpften Zustand der Puzzle-Teile Durchbrüche durch die Plattenebene aufweist.

10. Gehörknöchelchenprothese nach einem der Ansprüche 1 bis 8, **dadurch gekennzeichnet, dass** das plattenförmige erste Befestigungselement (81) im zusammengeknöpften Zustand der Puzzle-Teile eine in sich geschlossene Fläche bildet und keine Durchbrüche durch die Plattenebene aufweist.

11. Gehörknöchelchenprothese nach einem der vorhergehenden Ansprüche, **dadurch gekennzeichnet, dass** die radial äußeren Abschnitte (16; 26; 36, 46; 56; 66; 76; 96) des plattenförmigen ersten Befestigungselements (11; 21; 31; 41; 51; 61; 71; 91) einen äußeren Ringbereich bilden.

12. Gehörknöchelchenprothese nach Anspruch 11, **dadurch gekennzeichnet, dass** der radial äußere Ringbereich des plattenförmigen ersten Befestigungselements (41) in der Plattenebene eine radial nach außen verlaufende Ausbuchtung (47) aufweist.

13. Gehörknöchelchenprothese nach Anspruch 11, **dadurch gekennzeichnet, dass** der radial äußere Ringbereich des ersten Befestigungselements (61) eine einseitige Einbuchtung (67) zur Aufnahme des Hammergriffs aufweist.

14. Gehörknöchelchenprothese nach einem der vorhergehenden Ansprüche, **dadurch gekennzeichnet, dass** jedes Stegelement mit mindestens zwei anderen Stegelementen verbunden ist.

15. Gehörknöchelchenprothese nach einem der vorhergehenden Ansprüche, **dadurch gekennzeichnet, dass** der radial innere Ankoppelbereich (14; 24; 34; 44; 54; 64; 74; 84; 94) um den Flächenschwerpunkt des plattenförmigen ersten Befestigungselements (11; 21; 31; 41; 51; 61; 71; 81; 91) zentral angeordnet ist.

## Claims

1. An ossicular prosthesis (10; 20; 30; 40; 50; 60; 70; 80; 90), which replaces or spans at least one element or parts of an element of the ossicular chain, the ossicular prosthesis (10; 20; 30; 40; 50; 60; 70; 80; 90) comprising at one end a substantially plate-shaped first fastening element (11; 21; 31; 41; 51; 61; 71; 81; 91) for placing against the eardrum or against the footplate of the stirrup, and at its other end a second fastening element (12; 22; 32; 92) for mechanical connection to an element or parts of an element of the ossicular chain or to the inner ear, as well as a connecting element (13; 23; 93) joining together the two fastening elements (11,12; 21,22; 31,32; 41,12; 51,12; 61,12; 71,32; 81,12; 91,92) so as to conduct sound, and the plate-shaped first fastening element (11; 21; 31; 41; 51; 61; 71; 81; 91) having a radially inner coupling region (14; 24; 34; 44; 54; 64; 74; 84; 94) for the mechanical attachment of the first fastening element (11; 21; 31; 41; 51; 61; 71; 81; 91) to the connecting element (13; 23; 93), and a plurality of web elements (15,15',15"; 25,25',25"; 35,35',35",35"'; 45,45'; 55,55',55"; 65,65'; 75,75',75"; 85,85'; 95,95') for radial connection of the radially inner coupling region (14; 24; 34; 44; 54; 64; 74; 84; 94) to radially outer portions (16,16'; 26,26'; 36,36'; 46,46'; 56,56'; 66,66'; 76,76'; 86,86'; 96,96') of the first fastening element (11; 21; 31; 41; 51; 61; 71; 81; 91),
**characterized in that**
the coupling region (14; 24; 34; 44; 54; 64; 74; 84; 94) and/or web elements (15,15',15"; 25,25',25"; 35,35',35",35"'; 45,45'; 55,55',55"; 65,65'; 75,75',75"; 85,85'; 95,95') and/or radially outer portions (16,16'; 26,26'; 36,36'; 46,46'; 56,56'; 66,66'; 76,76'; 86,86'; 96,96') are geometrically configured in such a way that together they form parts of a jigsaw puzzle from which the plate-shaped first fastening element (11; 21; 31; 41; 51; 61; 71; 81; 91) can be fully assembled, in which case the individual pieces of the jigsaw can be interlinked or taken apart and the first fastening element (11; 21; 31; 41; 51; 61; 71; 81; 91) is inherently mechanically stable when the jigsaw-like pieces have been fitted together.

2. An ossicular prosthesis according to Claim 1, **characterized in that** both fastening elements (21, 22) are plate-shaped and are constructed from jigsaw-like pieces, the first fastening element (21) being formed as a flat headplate for mechanical connection to the eardrum and the second fastening element (22) being formed for placing of the ossicular prosthesis (20) on the footplate of the stirrup.

3. An ossicular prosthesis according to one of the preceding claims, **characterized in that** at least one radially inner jigsaw piece (16; 26) is provided which includes the coupling region (14; 24), **and in that**, in the assembled state, at least one radially outer jigsaw piece (16'; 26') encircles the radially inner jigsaw piece (16; 26).

4. An ossicular prosthesis according to one of the preceding claims, **characterized in that** at least one jigsaw piece (36'; 46'; 56'; 66', 76'; 86'; 96') is provided which, at the outer edge of the plate-shaped first fastening element (31; 41; 51; 61; 71; 81; 91), can be fitted in the plane of the plate from the side to at least one other jigsaw piece (36; 46; 56; 66; 76; 86; 96).

5. An ossicular prosthesis according to Claim 4, **characterized in that** the piece of the jigsaw (36'; 46'; 56'; 66'; 76') which can be fitted from the side is formed as appendix for the malleus or manubrium of the ossicular prosthesis (30; 40; 50; 60; 70) and, in the assembled state, projects sharply outwards in radial direction from the edge of the plate-shaped first fastening element (31; 41; 51; 61; 71).

6. An ossicular prosthesis according to Claim 4 or 5, **characterized in that** the jigsaw piece (56'; 66'; 76') which can be fitted from the side is resiliently anchored into the first fastening element (51; 61; 71).

7. An ossicular prosthesis according to Claim 6, **characterized in that** the jigsaw piece (66'; 76') which can be fitted from the side is anchored into the first fastening element (61; 71) by means of a clip or clamping element.

8. An ossicular prosthesis according to one of Claims 4 to 7, **characterized in that** the jigsaw piece (76') which can be fitted from the side is anchored with a snap-in action into the first fastening element (71), in particular by means of barbs (77).

9. An ossicular prosthesis according to one of Claims 1 to 8, **characterized in that**, in the interlinked state of the jigsaw pieces, the plate-shaped first fastening element (11; 21; 31; 41; 51; 61; 71; 91) has openings through the plane of the plate.

10. An ossicular prosthesis according to one of Claims 1 to 8, **characterized in that**, in the assembled state of the jigsaw pieces, the plate-shaped first fastening element (81) forms a coherent surface and has no openings through the plane of the plate.

11. An ossicular prosthesis according to one of the preceding claims, **characterized in that** the radially outer portions (16; 26; 36; 46; 56; 66; 76; 96) of the plate-shaped first fastening element (11; 21; 31; 41; 51; 61; 71; 91) form an outer annular region.

12. An ossicular prosthesis according to Claim 11, **characterized in that** the radially outer annular region of the plate-shaped first fastening element (41) has, in the plane of the plate, an area (47) bulging outwards in radial direction.

13. An ossicular prosthesis according to Claim 11, **characterized in that** the radially outer annular region of the first fastening element (61) has a recess (67) at one side for receiving the manubrium.

14. An ossicular prosthesis according to one of the preceding claims, **characterized in that** each web element is connected to at least two other web elements.

15. An ossicular prosthesis according to one of the preceding claims, **characterized in that** the radially inner coupling region (14; 24; 34; 44; 54; 64; 74; 84; 94) is centrally disposed about the centre of gravity of the surface of the plate-shaped first fastening element (11; 21; 31; 41; 51; 61; 71; 81; 91).

## Revendications

1. Prothèse pour osselets de l'oreille (10 ; 20 ; 30 ; 40 ; 50 ; 60 ; 70 ; 80 ; 90), qui remplace ou ponte au moins un élément ou des parties d'un élément de la chaîne des osselets de l'oreille, dans laquelle ladite prothèse (10 ; 20 ; 30 ; 40 ; 50 ; 60 ; 70 ; 80 ; 90) comprend, à une de ses extrémités, un premier élément de fixation (11 ; 21 ; 31 ; 41 ; 51 ; 61 ; 71 ; 81 ; 91) sensiblement en forme de plaque pour s'appuyer sur la membrane tympanique ou sur la plaque de base de l'étrier et, à son autre extrémité, un second élément de fixation (12 ; 22 ; 32 ; 92) pour se raccorder mécaniquement à un élément ou à des parties d'un élément de la chaîne des osselets de l'oreille ou à l'oreille interne, ainsi qu'un élément de raccordement (13 ; 23 ; 93) reliant les deux éléments de fixation (11,12 ; 21,22 ; 31,32 ; 41,12 ; 51,12 ; 61,12 ; 71,32 ; 81,12 ; 91,92) l'un à l'autre de manière à conduire le son, et dans laquelle le premier élément de fixation (11 ; 21 ; 31 ; 41 ; 51 ; 61 ; 71 ; 81 ; 91) en forme de plaque présente une zone de couplage radialement interne (14 ; 24 ; 34 ; 44 ; 54 ; 64 ; 74 ; 84 ; 94) pour le couplage mécanique du premier élément de fixation (11 ; 21 ; 31 ; 41 ; 51 ; 61 ; 71 ; 81 ; 91) sur l'élément de raccordement (13 ; 23 ; 93), ainsi que plusieurs traverses (15,15',15" ; 25,25',25" ; 35,35',35",35"' ; 45,45' ; 55,55',55" ; 65,65' ; 75,75',75" ; 85,85' ; 95,95') pour le raccordement radial de la zone de couplage radialement interne (14 ; 24 ; 34 ; 44 ; 54 ; 64 ; 74 ; 84 ; 94) avec des sections radialement externes (16,16' ; 26,26' ; 36,36' ; 46,46' ; 56,56' ; 66,66' ; 76,76' ; 86,86' ; 96,96') du premier élément de fixation (11 ; 21 ; 31 ; 41 ; 51 ; 61 ; 71 ; 81 ; 91),
**caractérisée en ce que**
la zone de couplage (14 ; 24 ; 34 ; 44 ; 54 ; 64 ; 74 ; 84 ; 94) et/ou les traverses (15,15',15" ; 25,25',25" ; 35,35',35", 35"' ; 45,45' ; 55,55',55" ; 65,65' ; 75,75',75" ; 85,85' ; 95,95') et/ou les sections radialement externes (16,16' ; 26,26' ; 36,36' ; 46,46' ; 56,56' ; 66,66' ; 76,76' ; 86,86' ; 96,96') est ou sont conçues géométriquement de manière à former ensemble des pièces d'un puzzle, à partir desquelles peut être complètement assemblé le premier élément de fixation (11 ; 21 ; 31 ; 41 ; 51 ; 61 ; 71 ; 81 ; 91) en forme de plaque, les pièces de puzzle individuelles pouvant être jointes l'une à l'autre ou être détachées l'une de l'autre par pression et le premier élément de fixation (11 ; 21 ; 31 ; 41 ; 51 ; 61 ; 71 ; 81 ; 91) étant mécaniquement stable en soi à l'état assemblé par pression.

2. Prothèse pour osselets de l'oreille selon la revendication 1, **caractérisée en ce que** les deux éléments de fixation (21, 22) sont conformés en plaque et constitués de pièces de puzzle, le premier élément de fixation (21) se présentant sous la forme d'une plaque de tête plane pour le raccordement mécanique avec la membrane tympanique, tandis que le second élément de fixation (22) est conformé pour appliquer ladite prothèse (20) sur la plaque de base de l'étrier.

3. Prothèse pour osselets de l'oreille selon l'une quelconque des revendications précédentes, **caractérisée en ce qu'**il est prévu au moins une pièce de puzzle radialement interne (16 ; 26) qui contient la zone de couplage (14; 24) et **en ce qu'**au moins une pièce de puzzle radialement externe (16'; 26') entoure en forme d'anneau la pièce de puzzle radialement interne (16; 26) à l'état assemblé par pression.

4. Prothèse pour osselets de l'oreille selon l'une quelconque des revendications précédentes, **caractérisée en ce qu'**il est prévu au moins une pièce de puzzle (36' ; 46' ; 56' ; 66' ; 76' ; 86' ; 96') qui peut être assemblée par pression, d'un côté, sur au moins une autre pièce de puzzle (36 ; 46 ; 56 ; 66 ; 76 ; 86 ; 96) sur le bord externe du premier élément de fixation (31 ; 41 ; 51 ; 61 ; 71 ; 81 ; 91) en forme de plaque dans le plan de la plaque.

5. Prothèse pour osselets de l'oreille selon la revendication 4, **caractérisée en ce que** la pièce de puzzle (36' ; 46' ; 56' ; 66' ; 76'), qui peut être assemblée par pression latéralement, est formée en appendice pour le marteau ou le manche de marteau de ladite prothèse (30 ; 40 ; 50 ; 60 ; 70) et, à l'état assemblé par pression, s'élève radialement vers l'extérieur en pointe depuis le bord du premier élément de fixation (31 ; 41 ; 51 ; 61 ; 71) en forme de plaque.

6. Prothèse pour osselets de l'oreille selon la revendication 4 ou 5, **caractérisée en ce que** la pièce de puzzle (56' ; 66' ; 76') qui peut être assemblée par pression latéralement est ancrée de manière élastique dans le premier élément de fixation (51 ; 61 ; 71).

7. Prothèse pour osselets de l'oreille selon la revendication 6, **caractérisée en ce que** la pièce de puzzle (66' ; 76') qui peut être assemblée par pression latéralement est ancrée au moyen d'un élément d'agrafage dans le premier élément de fixation (61 ; 71).

8. Prothèse pour osselets de l'oreille selon l'une quelconque des revendications 4 à 7, **caractérisée en ce que** la pièce de puzzle (76'), qui peut être assemblée par pression latéralement, est ancrée de manière encliquetable dans le premier élément de fixation (71), en particulier au moyen de barbes (77).

9. Prothèse pour osselets de l'oreille selon l'une quelconque des revendications 1 à 8, **caractérisée en ce que** le premier élément de fixation (11 ; 21 ; 31 ; 41 ; 51 ; 61 ; 71 ; 91) en forme de plaque présente, à l'état assemblé par pression des pièces de puzzle, des ouvertures à travers le plan de la plaque.

10. Prothèse pour osselets de l'oreille selon l'une quelconque des revendications 1 à 8, **caractérisée en ce que** le premier élément de fixation (81) en forme de plaque forme, à l'état assemblé par pression des pièces de puzzle, une surface fermée en soi et ne présente aucune ouverture à travers le plan de la plaque.

11. Prothèse pour osselets de l'oreille selon l'une quelconque des revendications précédentes, **caractérisée en ce que** les sections radialement externes (16 ; 26 ; 36 ; 46 ; 56 ; 66 ; 76 ; 96) du premier élément de fixation (11 ; 21 ; 31 ; 41 ; 51 ; 61 ; 71 ; 91) en forme de plaque forment une zone annulaire externe.

12. Prothèse pour osselets de l'oreille selon la revendication 11, **caractérisée en ce que** la zone annulaire radialement externe du premier élément de fixation (41) en forme de plaque présente, dans le plan de la plaque, un renflement (47) s'étendant radialement vers l'extérieur.

13. Prothèse pour osselets de l'oreille selon la revendication 11, **caractérisée en ce que** la zone annulaire radialement externe du premier élément de fixation (61) présente un renflement unilatéral (67) destiné à recevoir le manche du marteau.

14. Prothèse pour osselets de l'oreille selon l'une quelconque des revendications précédentes, **caractérisée en ce que** chaque traverse est raccordée à au moins deux autres traverses.

15. Prothèse pour osselets de l'oreille selon l'une quelconque des revendications précédentes, **caractérisée en ce que** la zone de couplage radialement interne (14 ; 24 ; 34 ; 44 ; 54 ; 64 ; 74 ; 84 ; 94) est aménagée centralement autour du centre de gravité surfacique du premier élément de fixation (11 ; 21 ; 31 ; 41 ; 51 ; 61 ; 71 ; 81 ; 91) en forme de plaque.
